# EUROPEAN PATENT SPECIFICATION

(11) **EP 0 460 327 B1**
(45) Date of publication and mention of the grant of the patent: **18.05.1994**
(21) Application number: 90313748.7
(22) Date of filing: 17.12.1990
(51) Int. Cl.: A61M 37/00

(54) **Medical capsule and apparatus for activating the same**
Kapsel für medizinische Zwecke und Vorrichtung zu deren Aktivierung
Capsule à usage médical et appareil pour l'activer

(30) Priority: 06.07.1990 JP 71511/90 U; 06.07.1990 JP 71512/90 U
(43) Date of publication of application: 11.12.1991
(73) Proprietor: MIYARISAN KABUSHIKI KAISHA, Hanishina-gun, Nagano-ken (JP)
(72) Inventor: Yuda, Shunichi, Miyarisan Kabushiki Kaisha, Hanishina-gun, Nagano-ken (JP); Ito, Hiroshi, Miyarisan Kabushiki Kaisha, Hanishina-gun, Nagano-ken (JP); Tanaka, Mamoru, Miyarisan Kabushiki Kaisha, Hanishina-gun, Nagano-ken (JP)
(74) Representative: Copp, David Christopher

(56) References cited:
- CH-A- 337 989
- DE-A- 3 339 323
- JP-A-52 128 677
- US-A- 3 396 726
- US-A- 4 457 752
- ELECTRONICS INTERNATIONAL vol. 49, no. 10, May 13, 1976, NEW-YORK US pages 3 - 4; "TELEMETER SAMPLES INTESTINAL LIQUIDS"

## Description

### BACKGROUND OF THE INVENTION

### 1. Field of the Invention

The present invention relates to a medical capsule for conducting sampling of a humor or dosage of a medicine at a desired location inside a living body.

### 2. Description of the Prior Art

Hitherto, a medical capsule has been known as a device which conducts sampling of, for example, intestinal liquid, or dosage of a medicine at a predetermined location in a living body, for the purpose of diagnosis or medical treatment.

The medical capsule has a capsule-like hermetic casing having an openable portion. When such a medical capsule swallowed by a patient has reached a predetermined position inside the patient's body, the openable portion is opened so as to sample a humor such as a gastric juice or to dose a medicine charged in the capsule. Then, the capsule is discharged through anus of the patient and collected. Such a medical capsule is disclosed, for example, in Japanese Patent Publication Nos. 53-53182, 55-9033. 55-49853, 57-1255, 57-39776 and 61-11104.

This medical capsule is very small and can easily swallow without any pain and, hence, is attracting attention in the medical fields. In particular, the medical capsule, when used for the purpose of dosage, can directly dose the medicine to the affected part of the body, thus attaining a great medical treating effect. For instance, when this capsule is used against a cancer in an alimentary system, a remarkable carcinostatic effect without side-effect, thus offering a desired treating effect.

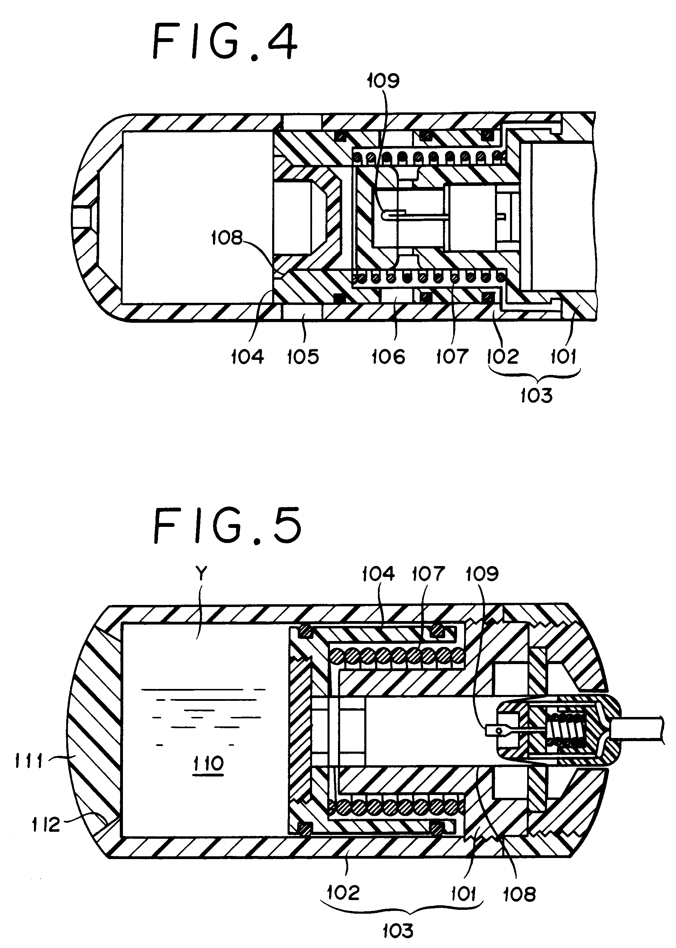

A known medical capsule for sampling will be described in more detail. As shown in Fig. 4, a medical capsule used for the sampling purpose has a hermetic casing 103 composed of a frame 101 and an outer cylinder 102. A piston 104 is movably received in the outer cylinder 102. As the piston 104 is moved, a vacuum region is produced in the outer cylinder 102 so that a gastric juice around the casing is sucked into the capsule through ports 105, 106, whereby the sampling of the gastric juice is conducted. This medical capsule for sampling is disclosed in Japanese Utility Model Publication No. 57-57684.

The piston 104 in this medical capsule operates by the springing force of a spring 107. Initially, the spring 107 is compressed and held in the compressed state by a fixing thread 108. After the capsule is swallowed by a patient, the position of the capsule is traced and monitored by an external monitoring device such as a roentgen apparatus. When the capsule has reached a predetermined position inside the body, a resonance of a resonance circuit is caused by an externally given radio wave so as to supply an electrical current to a filament 109. The fixing thread 108 is then molten by the heat generated by the filament 109 so that the piston 104 is projected by the resilient force of the spring 107, whereby the capsule sucks a gastric juice to complete the sampling.

Fig. 5 illustrates a known medical capsule used for the purpose of dosage. This capsule has a medicine chamber 110 in one end of which a piston 104 is received slidably, whereas a discharge port 112 formed in the other end of the medicine chamber 110 is normally closed by a plug 111. This medical capsule is disclosed, for example, in Japanese Patent Publication No. 55-49853 or 52-128677. Other portions are materially the same as those of the medical capsule shown in Fig. 4. For information, the same reference numerals are used to denote the same parts as those appearing in Fig. 4 and the description of such parts is omitted herein.

In operation, the fixing thread 108 is cut by the same method as that described before, so that the piston 104 moves inside the outer cylinder 102 so that the medicine Y in the medicine chamber is pressurized to forcibly remove the plug 111 so as to be discharged from the capsule.

Various other methods have been proposed for externally activating medical capsules. For instance, the following four methods have been proposed.

### (1) Direct remote control method

Long electric cords are connected to the capsule and the ends of the cords are held outside the body after the capsule is swallowed. When the capsule has reached a desired position, electrical current is supplied from an external power supply to the capsule through the cords so as to cut the fixing thread. Thus, the fixing thread is cut directly by a remote control.

### (2) Melting method

This method eliminates the necessity for external monitoring apparatus and external power supply. Namely, a portion of the hermetic casing is formed of a digestive film which can be molten by a predetermined environment inside the body. A cutter for cutting the thread is operated as this digestive film is molten. This method is disclosed in Japanese Patent Publication No. 55-30385.

### (3) Vibration method

A vibrator element inside the capsule is vibrated by an externally given vibratory energy so as to mechanically cut the fixing thread. This method is disclosed in Japanese Patent Publication No. 55-30386.

### (4) Supersonic method

In this method, a heater is energized by an externally applied supersonic wave so as to cut the fixing thread. This method is disclosed in Japanese Patent Publication No. 57-2015.

The term "medicine" in this specification is used to mean medical agents, microorganisms and so forth which are used for the purpose of diagnosis, curing, treatment and prophylaxis. The term "dosage" means application or dosage of such a medicine.

The following problem is encountered with the medical capsule of the type which supplies electrical current to a filament by a resonance circuit operated by an external instruction radio wave. Namely, in this type of medical capsule, an electrical current is continuously supplied even when the capsule is not being used once the capsule is assembled, in order to make sure of the safe operation of the capsule, and the switching of the circuit is conducted by means of a transistor. Consequently, the battery power is consumed heavily. In addition, a capsule which has been shelved long may fail to operate due to exhaustion of the battery. Furthermore, the effective reach of the radio wave, i.e., the distance within which the capsule can be operated without fail by the radio wave, differs according to individual patient. Usually, the effective reach of the radio wave is set to be 6 to 10 cm. The capsule therefore may not operate when applied to a fatty patient.

The direct remote control method also involves a problem in that it causes a physical pain on the patient because the patient is obliged to swallow not only the capsule but also electric cords connected thereto. From this point of view, indirect activating methods are preferred. Indirect activating methods, however, are also disadvantageous in that uncertain time lag tends to be caused between the moment at which the external activating apparatus is operated and the moment at which capsule is actually activated.

The medical capsule of digest type also contains a difficulty in that the control of position inside the body at which the capsule is activated is very difficult to conduct. The position varies according to the individual patients so that the discharge of the medicine cannot be controlled accurately.

The medical capsule of the vibration type also is defective due to inaccurate cutting of the fixing thread, thus impairing reliability of the dosage. In addition, this type of medical capsule has a complicated construction which makes it difficult to reduce the size and cost.

Furthermore, in known medical capsules of the type employing a closure plug, it is necessary that the closure plug securely holds the sealing condition when the capsule is not activated, whereas, when the capsule is activated, the plug 111 opens the discharge opening without fail and yet still held on the capsule without being severed therefrom. Consequently, a complicated connecting construction is required for connecting the plug 111 to the outer cylinder 102. In addition, the discharge of the medicine Y cannot be conducted with a high degree of reliability.

The fixing thread 108, which is used in the known medical capsule, is usually a cotton thread or a thread produced from synthetic fibers. Such a thread cannot be cut instantaneously. Namely, there is a slight time lag or delay until the fixing thread is cut. Such a delay makes it difficult to accurately control the position at which the capsule is activated, with the result that the expected curing or treating effect cannot be attained or the accuracy of examination is impaired due to inferior sampling.

### SUMMARY OF THE INVENTION

Accordingly, a first object of the present invention is to provide a medical capsule which can operate with reduced power consumption and which can be shelved for a long time and yet capable of operating without fail for dosage or sampling while minimizing pain posed on the patient, thereby overcoming the above-described problems of the prior art.

A second object of the present invention is to provide a fixing thread which can be cut easily when the capsule is used.

To achieve the first object, the invention provides a medical capsule including: a main block; an outer cylinder and a cover which are secured to both ends of the main block; a working chamber defined between the main block and the outer cylinder; a piston slidably received in the working chamber; a spring member acting between the piston and the main block so as to urge the piston towards the outer end of the outer cylinder; a fixing thread for fixing said piston to the main block against the force of the spring member; a filament for heating and melt-cutting the fixing thread; a battery for supplying electrical power to the filament; and a remote-controllable means capable of connecting the filament to the battery; wherein the improvement comprises: a medicine discharge opening formed in one end of the outer cylinder; a seal film closing the discharge opening so as to seal the working chamber; and a projection provided on the end of the piston 5 adjacent to the seal film so as to puncture the seal film when the piston advances.

To achieve the first object, the present invention also provides a medical capsule of the type described above, wherein the working chamber is a hollow chamber and is provided with a port through which a humor is sucked into the working chamber as a negative pressure is established in the working chamber as a result of the movement of the piston.

To achieve the first object, the present invention also provides a medical capsule of the type described above, wherein the remote-controllable means includes a normally-opened lead switch adapted to be turned on and off by an externally applied magnetic force.

Preferably, the seal film is made of polytetrafluoroethylene.

To achieve the second object, the present invention provides a medical capsule, wherein a combustible material is applied to the fixing thread.

When the medical capsule of the present invention is used, the position of the swallowed capsule in the patient's body is monitored from the exterior and, when the capsule has reached a predetermined position, an activating signal is applied externally so as to instantaneously cut the thread. In consequence, the piston is made to slide in the working chamber so that a projecting portion of the piston breaks the seal film so that whole of the medicine charged in the working chamber is discharged without fail.

The medical capsule of the present invention, regardless of whether it is used for dosing or sampling purpose, may include a magnetically operable lead switch as control means. Such a lead switch can be turned on easily and without fail by an external magnetic flux generator. It is therefore possible to activate the capsule at a desired position without fail. Furthermore, since the effective distance of activation can be increased, the medical capsule can be shelved for a longer time with reduced consumption of the battery power. In addition, any unfavorable effect is eliminated and the construction is simplified.

The fixing thread may be impregnated with a combustible material. When the filament is energized, the fixing thread is molten by the heat generated by the filament and by the heat produced by burning of the combustible material ignited by the heat generated by the filament. Consequently, the fixing thread can be cut almost instantaneously. Consequently, the operation of the piston can be effected without substantial delay after the operation of the external activating apparatus, thus enhancing the accuracy of the control of the capsule. The medical capsule of this type is used in a living body in which oxygen concentration is usually small. It is therefore preferred that the fixing thread is disposed in the cavity of the main block in contact with the filament, so that the melt-cutting of the fixing thread is promoted by burning of the combustible material by virtue of a certain amount of the oxygen contained in this cavity. The strength of the fixing thread itself is enhanced when the impregnating material is a combustible oil, whereby two incompatible requirements, i.e., ease of cutting of the thread and stability of fixing of the piston, are simultaneously satisfied.

### BRIEF DESCRIPTION OF THE DRAWINGS

Fig. 1 is a sectional view of a first embodiment of the medical capsule in accordance with the present invention;
Fig. 2 is a perspective view of a lead switch incorporated in the first embodiment;
Fig. 3 is a sectional view illustrative of the operation of the embodiment shown in Fig. 1;
Fig. 4 and 5 are illustrations of critical positions of known medical capsules.

### DESCRIPTION OF THE PREFERRED EMBODIMENTS

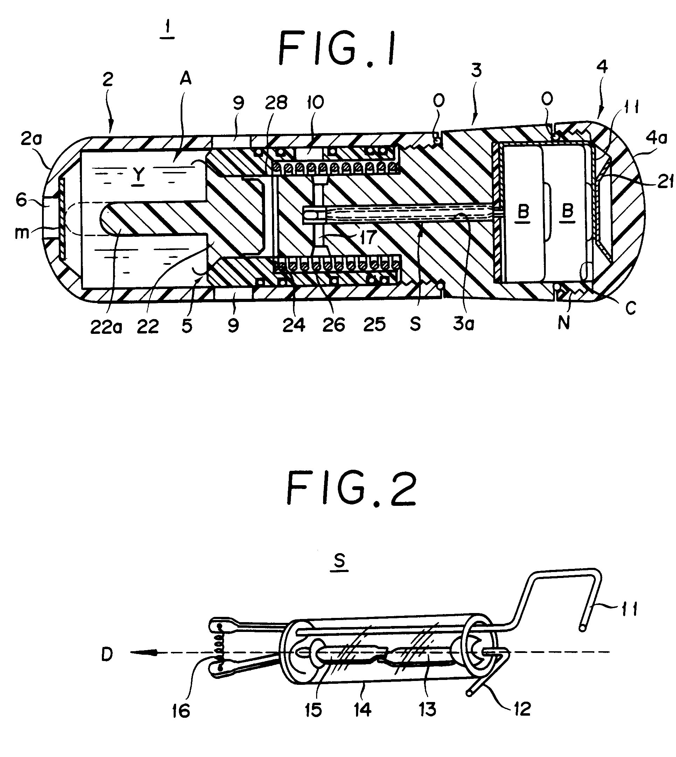

Fig. 1 shows a first embodiment of the medical capsule of the present invention intended for use in dosage of a medicine. The medical capsule of this embodiment has a generally elongated oval shape having a main block 3, an outer cylinder 2 made of polycarbonate and having a bottom-equipped cylindrical form, and a cover member 4, the outer cylinder 2 and the cover 4 being screwed to both ends of the main block 3 through intermediaries of "O" rings. A working chamber A is formed between the main block 3 and the outer cylinder 2. A piston 5 disposed in the working chamber A is urged towards the end of the outer cylinder 2 by means of the coiled spring 26 which acts between the piston 5 and the main block 3. A medicine chamber Y which is to be charged with a medicine is defined by the piston 5 and the outer cylinder 2.

Typical examples of the medicine charged in the medicine chamber Y are antibiotics formed from the following agents: antitherioma agents such as defcal (commercial name futrahool, produced by Taiho Yakuhin K.K.), anti-therioma agents (commercial name Picibanil, produced by Chugai Seiyaku K.K.), and Kawaratake-extract polysaccharide (commercial name Kurestin, produced by Kureha kagaku, K.K.); radioactive medicines such as sodium (commercial name Tekunesin Tc-99 produced by Medifix K.K.); sefem type medicines such as Sefarexin (commercial name Kefral, produced by Shionogi Seiyaku K.K.) and sefalexin (commercial name Keflex, produced by Shionogi Seiyaku K.K.); and synthetic penicillin type medicines such as sodium kuroxacillin (commercial name Orben, produced by Shionogi Seiyaku K.K.).

The outer cylinder 2 and the cover 4 respectively have dome-shaped bottoms 2a and 4a which enable the capsule to smoothly move in the patient's body. The bottom 2a of the outer cylinder 2 is provided with a medicine discharge port 6 which is usually covered with a seal film m. Preferably, the seal film m is made of a thin film of a material having a high resistance to medical liquid and easy to break, such as polytetrafluoroethylene. This, however, is only illustrative and the seal film m may be formed from other types of material.

Preferably, the outer cylinder 2 is provided with ports 9 formed substantially in the center of the side walls thereof. It is also preferred that ports 10 are formed in an intermediate portion of the piston 5. In operation, humor is introduced to the back side of the piston 5 through the ports 9 and 10 so as to negate any negative pressure behind the piston 5, thus ensuring smooth operation of the piston 5.

When pickup of the humor is to be avoided, these ports 9, 10 are omitted and, instead, compressed air is charged in a battery chamber C inside the cover 4. In such a case, the thread portion N where the cover 4 is screwed to the main block 3 has a substantial length so that air initially confined in the battery chamber C is progressively compressed as the cover 4 is screwed to the main block 3.

The main block 3 also is made of polycarbonate. The main block 3 is provided at its right end with a recess for accommodating a battery B and at its center with a central bore 3a which receives a lead switch s. The lead switch s is a normally-opened switch having two lead lines 11, 12 connected to a pair of silver batteries B, B (each being of 1.5V) as shown in Fig. 2. One 12 of the lead lines has one end connected to a contact 13 which is received in a glass tube 14 so as not to unintentionally contact with the other contact 15. The contact 15 and the lead line 11 are extended externally of the glass tube 14 and are connected to each other through a filament 16. The contacts 13 and 15 are resilient magnetic members and are highly sensitive to magnetic lines of force of a direction of an arrow D so as to be turned on in response to this magnetic lines of force, thereby allowing electrical power to be supplied from the batteries B to the filament 16 to generate heat. When no magnetic force is applied, these contacts are kept off, i.e., away from each other, due to their resiliency.

In the drawings, numeral 21 designates a spring for pressing the battery B.

The piston 5 is a cylindrical member made of polycarbonate and adapted to slide within the outer cylinder 2. A thread retaining cap 22 is detachably press-fitted in the end of the outer cylinder 2. A projection 22a for puncturing or piercing the seal film m is provided on the end of the cap 22. A coiled spring 26is loaded between a step 24 formed on the inner peripheral surface of the piston 5 and a step 25 formed on the main block 3.

The piston 5 is fixed to the main block 3 by a fixing thread 28 against the force of the coiled spring 26. Although not exclusive, the fixing thread 28 is made of a material having a high strength and a comparatively low melting point, e.g., a polyethylen terephthalate thread having a melting point ranging between 200°C and 270°C.

The fixing thread 28 has both ends fastened to the thread retaining cap 22 and is extended so as to contact with the filament 16 within a bore-like chamber 17 of a predetermined volume formed in the main block 3.

Preferably, a combustible material is applied to the fixing thread 28. The combustible material is preferably a non-volatile substance having a comparatively low flash point. Practical examples of the combustible material are wax, benzene, naphthalene and so forth, as well as combustible oil. The combustible material applied to the fixing thread 28 is easily ignited to promote the rupture of the fixing thread 28 by melting. When an oil is used as the combustible material, the mechanical strength of the fixing thread is increased,so that two generally incompatible requirements, i.e., ease of rupture and stability of support of the piston, are satisfied simultaneously.

The operation of the medical capsule and actuating apparatus of the invention will be described hereinunder.

The medical capsule for dosage of a medicine, set up in the manner illustrated in Fig. 1, is swallowed by a patient. The position of the capsule 1 inside the patient's body is traced and monitored by an external monitoring device such as an X-ray transmissive camera. When the capsule has reached a predetermined position inside the patient's body, the magnetic field generating means are started and are energized to generate magnetic fields, and the contacts 13 and 15 of the normally opened lead switches S in the capsule 1 inside the patient's body are brought into contact by the magnetic fields.

Thus, the contacts 13 and 15 of the normally opened lead switches s in the capsule 1 inside the patient's body M are brought into contact to close the circuit by the magnetic fields generated by the magnetic field generating units 51. Until these contacts 13 and 15 contact each other, the current from the battery B does not flow so that wasteful use of the battery power is prevented.

As a result of the closing of the circuit by the contacts 13, 15, electrical current is supplied to the filament 16 to red-heat the same, so that the fixing thread 28 fixing the piston 5 is molten and cut. Consequently, the piston 5 moves to the left as viewed in the drawings by the force of the coiled spring 26. In an initial period of the movement t of the piston 5, the ports 9 in the outer cylinder 2 and the ports 10 in the piston 5 partially overlap each other, so that the working chamber A is communicated with the exterior so as to allow a fluid such as humor to flow into the working chamber A through the ports 9 and 10. In the case where the ports 9 and 10 are not provided, the pressurized air confined in the battery chamber C cancels the negative back pressure behind the piston 5,thus ensuring a smooth operation of the piston.

The medicine in the medicine chamber is pressurized in the beginning period of movement of the piston 5. This pressure, however, is absorbed by an elastic deformation of the seal film m and, hence, does not produce any undesirable effect.

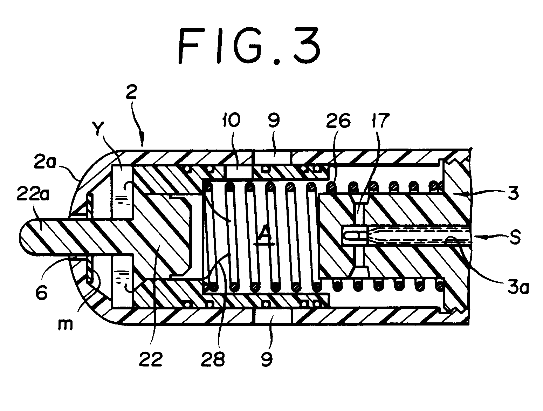

A further movement of the piston 5 causes the projection 22a on the piston 5 to break the seal film m as shown in Fig. 3, so that the whole of the medicine in the medicine chamber Y is discharged to the outside. Almost no portion of the medicine remains in the capsule because the spring continues to press the piston 5 even after the overlap of the ports 9 and 10 is ceased or even after the pressure of the compressed air has been reduced.

The capsule 1 after the dosage further moves in the patient's body and is discharge together with the human waste.

Furthermore, although capsules 1 for dosage have been described as the embodiment, it is possible to construct a single medical capsule which can simultaneously perform both dosage and sampling.

## Claims

1. A medical capsule including: a main block (3); an outer cylinder (2) and a cover (4) which are secured to opposite ends of said main block (3); a working chamber (A) defined between the main block (3) and the outer cylinder (2); a piston (5) slidably received in the working chamber (A): a spring member acting between said piston (5) and said main block (3) so as to urge said piston (5) towards the outer end of said outer cylinder; a fixing thread (28) for fixing said piston (5) of said main block (3) against the force of said spring member (26); a filament (16) for heating and melt-cutting said fixing thread (28); a battery (B) for supplying electrical power to said filament (16); and a remote-controllable means (S) capable of connecting said filament (16) to said battery (B); a medicine discharge opening (6) is formed in one end of said outer cylinder (2); Characterized in that a seal film (m) closes said discharge opening (6) so as to seal said working chamber (A); and a projection (22a) is provided on the end of said piston (5) adjacent to said seal film (m) so as to puncture said seal film (m) when said piston (5) advances.

2. A medical capsule according to Claim 1, wherein said working chamber (A) is a hollow chamber and is provided with a port through which a humor is sucked into said working chamber (A) as a negative pressure is established in said working chamber (A) as a result of the movement of said piston.

3. A medical capsule according to Claim 1, wherein said remote-controllable means (S) includes a normally-opened lead switch adapted to be turned on and off by an externally applied magnetic force.

4. A medical capsule according to Claim 3, wherein said lead switch (S) has a pair of contacts (13) and (15) made from a resilient magnetic material.

5. A medical capsule according to Claim 1, wherein said seal film (m) is made of polytetrafluoroethylene.

6. A medical capsule according to Claim 1, wherein a combustible material is applied to said fixing thread (28).

7. A medical capsule according to Claim 6, wherein said fixing thread is provided so as to contact said filament (16) in a cavity (17) formed in said main block (3).

8. A medical capsule according to Claim 6, wherein said fixing thread (28) is made of polyethylene terephthalate and is impregnated with a combustible oil.

9. A medical capsule according to Claim 1, wherein said block (3), outer cylinder (2) and said cover (4) are made from a polycarbonate resin.

10. A medical capsule according to Claim 1, wherein said battery (B) is a silver battery.

## Patentansprüche

1. Medizinische Kapsel mit einem Hauptblock (3); einem Außenzylinder (2) und einem Deckel (4), die an den gegenüberliegenden Enden des Hauptblockes (3) befestigt sind; einer Arbeitskammer (A), die zwischen dem Hauptblock (3) und dem Außenzylinder (2) ausgebildet ist; einem Kolben (5), der in der Arbeitskammer (A) gleitbar aufgenommen ist, wobei ein Federglied zwischen diesem Kolben (5) und dem Hauptblock (3) derart wirkt, daß der Kolben (5) in Richtung auf das Außenende des Außenzylinders gedrückt wird; einem Fixierfaden (28) zur Fixierung dieses Kolbens (5) des Hauptblockes (3) gegenüber der durch das Federglied (26) ausgeübten Kraft; einem Heizfaden (16) zum Erhitzen und Schmelz-Schneiden des Fixierfadens (28), einer Batterie (B), welche die Energie für den Heizdraht (16) liefert; ein fernlenkbares Mittel (S) zum Verbinden des Heizdrahtes (16) mit der Batterie (B), und einer Auslaßöffnung (6) für eine Medizin in einem Ende des Außenzylinders (2),
dadurch **gekennzeichnet**,
daß ein Abdichtfilm (m) die Auslaßöffnung verschließt, um die Arbeitskammer (A) abzudichten, und ein dem Abdichtfilm (m) benachbarter Vorsprung (22a) an einem Ende des Kolbens (5) vorgesehen ist, so daß der Abdichtfilm (m) durchstochen wird, wenn sich der Kolben (5) vorwärtsbewegt.

2. Medizinische Kapsel nach Anspruch 1, bei der die Arbeitskammer (A) eine Hohlkammer ist und mit einem Einlaß ausgestattet ist, durch die eine Körperflüssigkeit in die Arbeitskammer (A) eingesaugt wird, sobald in dieser Arbeitskammer (A) als Folge der Bewegung des Kolbens ein Unterdruck erzeugt wird.

3. Medizinische Kapsel nach Anspruch 1, bei der das fernlenkbare Mittel (S) einen normalerweise geöffneten Schalter aufweist, der durch eine von außen ausgeübte magnetische Kraft an- und ausgeschaltet werden kann.

4. Medizinische Kapsel nach Anspruch 3, bei der der Schalter (S) ein Paar von Kontakten (13) und (15) aus einem federnden magnetischen Material aufweist.

5. Medizinische Kapsel nach Anspruch 1, bei der der Abdichtfilm (m) aus Polytetrafluorethylen besteht.

6. Medizinische Kapsel nach Anspruch 1, bei der der Fixierfaden (28) mit einem verbrennbaren Material ausgerüstet ist.

7. Medizinische Kapsel nach Anspruch 6, bei der der Fixierfaden derart ausgestattet ist, daß er den Heizdraht (16) in einem in dem Hauptblock (3) ausgebildeten Hohlraum (17) berührt.

8. Medizinische Kapsel nach Anspruch 6, bei der der Fixierfaden (28) aus Polyethylenterephthalat besteht und mit einem verbrennbaren Öl getränkt ist.

9. Medizinische Kapsel nach Anspruch 1, bei der der Block (3), der Außenzylinder (2) und der Deckel (4) aus einem Polycarbonatharz bestehen.

10. Medizinische Kapsel nach Anspruch 1, bei der die Batterie (B) eine Silberbatterie ist.

## Revendications

1. Capsule médicale comprenant un corps principal (3), un cylindre extérieur (2) et un couvercle (4) qui sont fixés aux extrémités opposées dudit corps principal (3), une chambre de travail (A) définie entre le corps principal (3) et le cylindre extérieur (2), un piston (5) logé de façon coulissante dans la chambre de travail (A), un élément formant ressort agissant entre ledit piston (5) et ledit corps principal (3) pour pousser ledit piston (5) vers l'extrémité extérieure dudit cylindre extérieur, un filetage de fixation (28) pour fixer ledit piston (5) dudit corps principal (3) à l'encontre de la force dudit élément formant ressort (26), un filament (16) pour chauffer et couper par fusion ledit filetage de fixation (28), une pile (B) pour alimenter ledit filament (16) en énergie électrique et un moyen commandable à distance (S) capable de connecter ledit filament (16) à ladite pile (B), un orifice (6) de distribution de médicament étant formé dans une extrémité dudit cylindre extérieur (2), caractérisée en ce qu'une pellicule étanche (m) ferme ledit orifice de distribution (6) pour sceller ladite chambre de travail (A) et une saillie (22a) est ménagée sur l'extrémité dudit piston (5), à proximité de ladite pellicule étanche (m), pour perforer ladite pellicule étanche (m) quand ledit piston (5) avance.

2. Capsule médicale selon la revendication 1, dans laquelle ladite chambre de travail (A) est une chambre creuse et est munie d'une embouchure par laquelle est aspirée une humeur dans la chambre de travail (A) lorsqu'une pression négative est établie dans ladite chambre de travail (A) en résultat du déplacement dudit piston.

3. Capsule médicale selon la revendication 1, dans laquelle ledit moyen commandable à distance (S) inclut un interrupteur électrique normalement ouvert apte à être ouvert et fermé par une force magnétique appliquée extérieurement.

4. Capsule médicale selon la revendication 3, dans laquelle ledit interrupteur électrique (S) comprend une paire de contacts (13) et (15) faits d'un matériau magnétique élastique.

5. Capsule médicale selon la revendication 1, dans laquelle ladite pellicule étanche (m) est faite de polytétrafluoréthylène.

6. Capsule médicale selon la revendication 1, dans laquelle un matériau combustible est appliqué sur ledit filetage de fixation (28).

7. Capsule médicale selon la revendication 6, dans laquelle ledit filetage de fixation est placé pour être en contact avec ledit filament (16) dans une cavité (17) formée dans ledit corps principal (3).

8. Capsule médicale selon la revendication 6, dans laquelle ledit filetage de fixation (28) est fait de poly(téréphthalate d'éthylène) et est imprégné d'une huile combustible.

9. Capsule médicale selon la revendication 1, dans laquelle lesdits corps (3), cylindre extérieur (2) et couvercle (4) sont faits d'une résine polycarbonate.

10. Capsule médicale selon la revendication 1, dans laquelle ladite pile (B) est une pile à l'argent.
